# EUROPEAN PATENT SPECIFICATION

(11) **EP 3 947 725 B1**
(45) Date of publication and mention of the grant of the patent: **06.09.2023**
(21) Application number: 20725731.2
(22) Date of filing: 26.03.2020
(51) Int. Cl.: C12Q 1/6823, C12Q 1/6834, C12Q 1/6825

(54) **APPARATUS, SYSTEM AND METHOD**
VORRICHTUNG, SYSTEM UND VERFAHREN
APPAREIL, SYSTÈME ET PROCÉDÉ

(30) Priority: 26.03.2019 GB 201904191
(43) Date of publication of application: 09.02.2022
(73) Proprietor: Nanovery Limited, Newcastle upon Tyne NE4 5BX (GB)
(72) Inventor: KOZYRA, Jerzy, Gateshead, NE8 2DF (GB); LEE, Mun Ching, London SE1 6AQ (GB)
(74) Representative: McShane, Andrea Renata
(86) International application number: PCT/GB2020/050809
(87) International publication number: WO 2020/193980

(56) References cited:
- WO-A2-2010/019414
- WANG HUA-FENG ET AL: "A versatile label-free electrochemical biosensor for circulating tumor DNA based on dual enzyme assisted multiple amplification strategy", BIOSENSORS AND BIOELECTRONICS, ELSEVIER SCIENCE LTD. UK, AMSTERDAM, NL, vol. 122, 8 September 2018 (2018-09-08), pages 224-230, XP085504842, ISSN: 0956-5663, DOI: 10.1016/J.BIOS.2018.09.028
- LUO JIE ET AL: "Enzyme-free amplified detection of circulating microRNA by making use of DNA circuits, a DNAzyme, and a catalytic hairpin assembly", MIKROCHIMICA ACTA, SPRINGER VERLAG, VIENNA, AT, vol. 185, no. 1, 8 December 2017 (2017-12-08), pages 1-6, XP036376093, ISSN: 0026-3672, DOI: 10.1007/S00604-017-2565-9 [retrieved on 2017-12-08]
- HU XIUXUE ET AL: "Single-molecule catalytic hairpin assembly for rapid and direct quantification of circulating miRNA biomarkers", ANALYTICA CHIMICA ACTA, ELSEVIER, AMSTERDAM, NL, vol. 1042, 22 August 2018 (2018-08-22), pages 109-115, XP085513462, ISSN: 0003-2670, DOI: 10.1016/J.ACA.2018.08.037
- Evelyn M. Linardy ET AL: "EzyAmp signal amplification cascade enables isothermal detection of nucleic acid and protein targets", Biosensors and Bioelectronics, vol. 75, 1 January 2016 (2016-01-01), pages 59-66, XP055399153, Amsterdam , NL ISSN: 0956-5663, DOI: 10.1016/j.bios.2015.08.021
- XIONG ERHU ET AL: "An ultrasensitive electrochemical immunoassay based on a proximity hybridization-triggered three-layer cascade signal amplification strategy", ANALYST, vol. 144, no. 2, 5 November 2018 (2018-11-05), pages 634-640, XP055954689, UK ISSN: 0003-2654, DOI: 10.1039/C8AN01800F Retrieved from the Internet: URL:https://pubs.rsc.org/en/content/articl epdf/2019/an/c8an01800f>

## Description

The present invention relates to a DNA nanostructure, and a method of producing a DNA nanostructure.

Circulating tumour deoxyribonucleic acid (ctDNA) is DNA circulating in the bloodstream that is specific to, and indicative, of a tumour and its state of progression. While this can give valuable information on the state of a tumour without the burden of taking a biopsy from a patient, the ctDNA in a blood sample poses a problem of scarce analyte in a complex sample. Blood samples also contain DNA that is not associated with tumours, cell-free DNA (cfDNA), so it is additionally necessary to distinguish ctDNA from such cfDNA. In order to detect trace amounts of DNA with both high specificity and high sensitivity new approaches are sought.

Wang Hua-Feng et al, "A versatile label-free electrochemical biosensor for circulating tumor DNA based on dual enzyme assisted multiple amplification strategy", Biosensors and Bioelectronics, vol. 122, September 2018, pages 224-230, describes a biosensor based on dual enzyme assisted multiple amplification strategy for detection of circulating tumor DNA (ctDNA). Luo Jie et al, "Enzyme-free amplified detection of circulating microRNA by making use of DNA circuits, a DNAzyme, and a catalytic hairpin assembly", Mikrochimica Acta, vol. 185, no. 1, December 2017, pages 1-6, describes a fluorometric assay for the determination of microRNA-182 based on the use of DNA circuits and DNAzyme. Hu Xiuxue et al, "Single-molecule catalytic hairpin assembly for rapid and direct quantification of circulating miRNA biomarkers", Analytica Chimica Acta, vol. 1042, August 2018, pages 109-115, describes a method for quantification of circulating miRNA in serum by combining dynamic DNA circuit and single molecule fluorescence detection. WO 2010/019414 A2 describes methods for detecting a target nucleic acid using an enzymatic amplification cascade of restriction endonucleases to detect nucleic acid. Evelyn M. Linardy et al, "EzyAmp signal amplification cascade enables isothermal detection of nucleic acid and protein targets", Biosensors and Bioelectronics, vol. 75, January 2016, pages 59-66, describes an isothermal signal amplification cascade called EzyAmp (enzymatic signal amplification) using an enzymatic amplification cascade involving restriction endonucleases. XIONG ERHU et al, "An ultrasensitive electrochemical immunoassay based on a proximity hybridization-triggered three-layer cascade signal amplification strategy", Analyst, vol. 144, no. 2, November 2018, pages 634-640, describes a three-layer cascade signal amplification involving an exonuclease III enzyme in the first layer, catalytic hairpin assembly in the second layer, and rolling circle amplification in the third layer.

According to a first aspect there is provided an ensemble of DNA formations for detecting a biomarker as defined by the claims.

By virtue of the ensemble of DNA sequences that form a pathway for detection, the functioning of the pathway can be engineered and controlled with precision. By combining detection, amplification and response very sensitive detection can be enabled.

Preferably two or more of the formations are attached to a common body. By thus collocating the formations efficient interaction between the formations can be enabled. Preferably the detector, at least part of the amplifier, and the responder are attached to the common body. The formations may be attached to a common body by way of a tether or an anchor. The tether may be adapted to enable different DNA formations to come into proximity to one another. Alternatively the DNA formations may be attached to the common body at an anchor portion of the DNA formation.

For versatility and to enable precise design and assembly the common body may be a DNA formation. For simplicity the common body may be a nanoparticle. For integration with further macroscopic features the common body may be a substrate. For controlling interactions two or more of the formations may be attached to the common body in a predetermined arrangement. For example, an amplifier may be positioned between a detector and a responder.

The detector may be adapted to accept a target DNA sequence and thereby to release a trigger DNA sequence. Detection of a target DNA sequence as a biomarker can permit very selective detection and provide high specificity. The target DNA sequence may be circulating tumour DNA. The detector may be adapted to accept a target RNA sequence (preferably circulating tumour RNA) and thereby to release a trigger DNA sequence. The detector may be adapted to accept a target protein (preferably a circulating tumour protein) and thereby to release a trigger DNA sequence.

For greater specificity the detector may be adapted for hybridisation with a plurality of biomarkers and thereby to release one or more trigger DNA sequences.

Preferably the detector comprises one or more probe DNA sequences attached (preferably in a predetermined arrangement, optionally by way of a tether) to a DNA body and adapted to hybridise with a target DNA sequence and release a trigger DNA sequence.

The probe DNA sequence may be configured to hybridise with the target DNA sequence and release the trigger DNA sequence by competitive hybridisation, preferably by toehold-mediated strand displacement or by toe-hold exchange. Preferably the probe DNA sequence is hybridised to the trigger DNA sequence and is adapted to hybridise preferentially with the target DNA sequence and thereby release the trigger DNA sequence.

For higher specificity and fewer false positives the ensemble of DNA formations may further comprise a blocker adapted to hybridise an interfering DNA sequence and optionally thereby to release a non-interacting DNA sequence. The interfering DNA sequence is preferably cell free DNA. The blocker may be a blocker DNA sequence configured to hybridise with the interfering DNA sequence and optionally release the non-interacting DNA sequence by competitive hybridisation, preferably by toe-hold-mediated strand displacement or by toe-hold exchange.

Preferably the amplifier comprises a plurality of amplifier subunits, each amplifier subunit adapted to hybridise a trigger DNA sequence and to release a key DNA sequence and the trigger DNA sequence or a further trigger DNA sequence. The further trigger DNA sequence preferably has the same DNA sequence as the trigger DNA sequence. The plurality of amplifier subunits are preferably functionally identical to one another, and preferably release copies of the same key DNA sequence. A plurality of first amplifier subunits may be adapted to release first key DNA sequences and a plurality of second amplifier subunits may be adapted to release second key DNA sequences.

The amplifier may comprise one or more instances of one or more amplifier DNA sequences (optionally anchored to a common body, preferably a DNA body, preferably in a predetermined arrangement, optionally by way of a tether) adapted to hybridise with a trigger DNA sequence; and one or more instances of a key holder DNA sequence (optionally anchored to a common body, preferably a DNA body, preferably in a predetermined arrangement, optionally by way of a tether) adapted to release a key DNA sequence.

A first amplifier DNA sequence may be adapted to change conformation upon hybridisation with the trigger DNA sequence. A second amplifier DNA sequence may be adapted to change conformation upon hybridisation with the first DNA sequence and thereby to release the trigger DNA sequence. The first amplifier DNA sequence may be configured to hybridise with the second amplifier DNA sequence and release the trigger DNA sequence by competitive hybridisation (preferably by toe-hold-mediated strand displacement or by toe-hold exchange) and/or one or more conformation change (preferably by a conformation change of the first amplifier DNA sequence and a conformation change of the second amplifier DNA sequence).

The key holder DNA sequence may be hybridised to the key DNA sequence. The key holder DNA sequence may be adapted to hybridise preferentially with the amplifier DNA sequence or a part thereof and thereby release the key DNA sequence, preferably by competitive hybridisation (preferably by toe-hold-mediated strand displacement or by toehold exchange) and/or conformation change (preferably by a conformation change of one or more of the amplifier DNA sequences).

For robustness and the avoidance of false positives the amplifier may comprise a plurality of amplification pathways for release of different species of key DNA sequences. A plurality of first amplifier subunits may be adapted to release first key DNA sequences and a plurality of second amplifier subunits may be adapted to release second key DNA sequences. For high amplification by way of a feed-forward loop the plurality of amplification pathways may be adapted to amplify each other.

Preferably the responder comprises a DNA capture formation adapted at least partially to enclose a reporter in a first configuration, and to release the reporter in a second configuration. This can enable conditional release.

For ease of production of a signal detectable to an observer the reporter may be a reporter gene with a promoter portion, and the DNA capture formation may be adapted at least partially to enclose the promoter portion in the first configuration.

The DNA capture formation may be adapted to change from the first configuration to the second configuration upon hybridisation of at least one key DNA sequence to at least one lock DNA sequence portion of the DNA capture formation. The lock DNA sequence may be configured to hybridise with the key DNA sequence by competitive hybridisation (preferably by toe-hold-mediated strand displacement or by toe-hold exchange).

According to another aspect there is provided a method of detecting a target biomarker comprising introducing an ensemble of DNA formations according to any preceding claim to a sample of bodily fluid, preferably blood, preferably in vitro.

According to another aspect there is provided a method of detecting a target biomarker as defined by the claims.

Hybridising the trigger DNA sequence to an amplifier DNA sequence and thereby causing one or more conformational changes of the amplifier DNA sequence, releasing a key DNA sequence, and releasing the or a trigger DNA sequence may be by way of a second amplifier DNA sequence adapted for hybridisation to the amplifier DNA sequence. Hybridising the key DNA sequence to a lock DNA sequence portion of a DNA capture formation and thereby cause a signal detectable to an observer to be produced may be by changing configuration of the DNA capture formation to release a reporter.

The amplifier DNA sequence may comprise one or more one or more instances of one or more amplifier DNA sequences adapted to hybridise with a trigger DNA sequence; and one or more instances of a key holder DNA sequence adapted to release a key DNA sequence.

The probe, amplifier DNA sequence and/or DNA capture formation may be as aforementioned. The method may use an ensemble of DNA formations as aforementioned.

The probe, amplifier DNA sequence, and/or DNA capture formation may be collocated on a common body, preferably a DNA structure.

The method may further comprise introducing the DNA structure to a sample of bodily fluid, preferably blood, preferably *in vitro.*

According to another aspect there is provided a computer program product comprising software code adapted to generate, when executed, a simulation of the ensemble of DNA formations as aforementioned.

According to another aspect there is provided a method of producing an ensemble of DNA formations as aforementioned comprising simulating the ensemble of DNA formations; determining, on the basis of the simulation, DNA sequences for the DNA formations; and producing the ensemble of DNA formations on the basis of the thereby determined DNA sequences.

As used herein the terms 'DNA sequence' and 'DNA strand' are preferably interchangeable, and preferably refer to a DNA strand having a given sequence.

As used herein the term 'amplification' preferably refers to a process where input of a number of instances of an input species results in release of a greater number of instances of an output species. An 'amplifier' preferably refers to a functional unit adapted to accept a number of instances of an input species and in result thereof release of a greater number of instances of an output species.

As used herein the term 'ensemble of DNA formations' preferably refers to a plurality of DNA formations in a common solution, attached to a common substrate, or attached to a common body. As used herein the term 'DNA formation' preferably refers to a DNA strand, preferably having a specific sequence and/or forming a specific structure. Such a DNA strand may be with or without a further DNA strand hybridised to it. Such a DNA strand may be in one of different possible conformations.

According to another aspect there is provided a computer program and a computer program product for carrying out any of the methods described herein and/or for embodying any of the apparatus features described herein. According to another aspect there is provided a non-transitory computer readable medium having stored thereon a program for carrying out any of the methods described herein and/or for embodying any of the apparatus features described herein. According to another aspect there is provided a computer program product comprising software code for carrying out any method as herein described. Features implemented in hardware may generally be implemented in software, and vice versa. Any reference to software and hardware features herein should be construed accordingly.

The invention also provides a signal embodying a computer program for carrying out any of the methods described herein and/or for embodying any of the apparatus features described herein, a method of transmitting such a signal, and a computer product having an operating system which supports a computer program for carrying out any of the methods described herein and/or for embodying any of the apparatus features described herein.

As used herein, means plus function features may be expressed alternatively in terms of their corresponding structure, such as a suitably programmed processor and associated memory.

These and other aspects of the present invention will become apparent from the following exemplary embodiments that are described with reference to the following figures in which:
Figure 1 is a schematic of a DNA nano device;
Figures 2a-c are diagrams of a detector portion of a DNA nano device in different states;
Figures 3a-d are diagrams of an amplifier portion of a DNA nano device in different states;
Figures 4a and 4b are diagrams of a responder portion of a DNA nano device in different states;
Figures 5a-d are diagrams of an alternative amplifier portion of a DNA nano device in different states;
Figure 6 is a diagram of another alternative amplifier portion of a DNA nano device;
Figure 7 is a diagram of a DNA nano device with a blocking portion;
Figure 8 is a diagram of a blocking portion of a DNA nano device; and

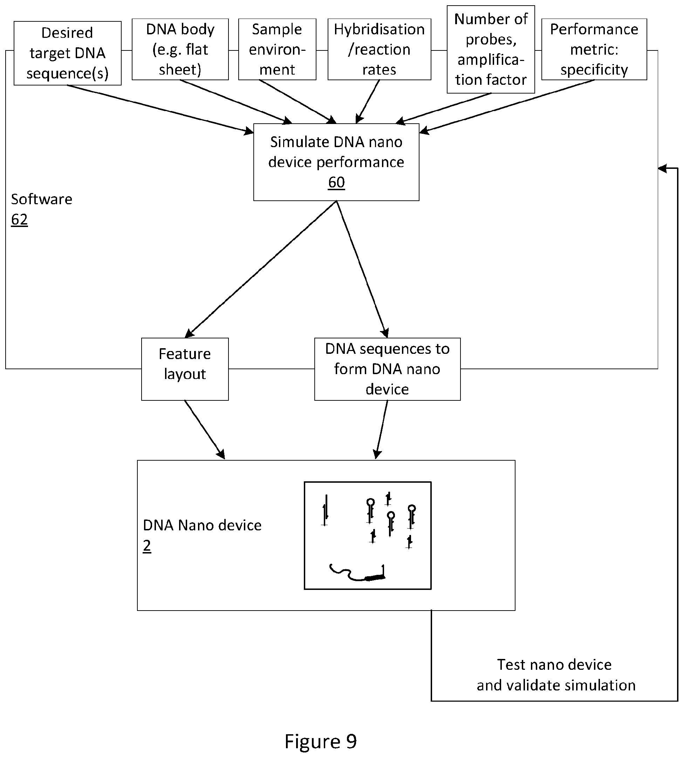

Figure 9 illustrates an example of a method of generating a DNA nano device.

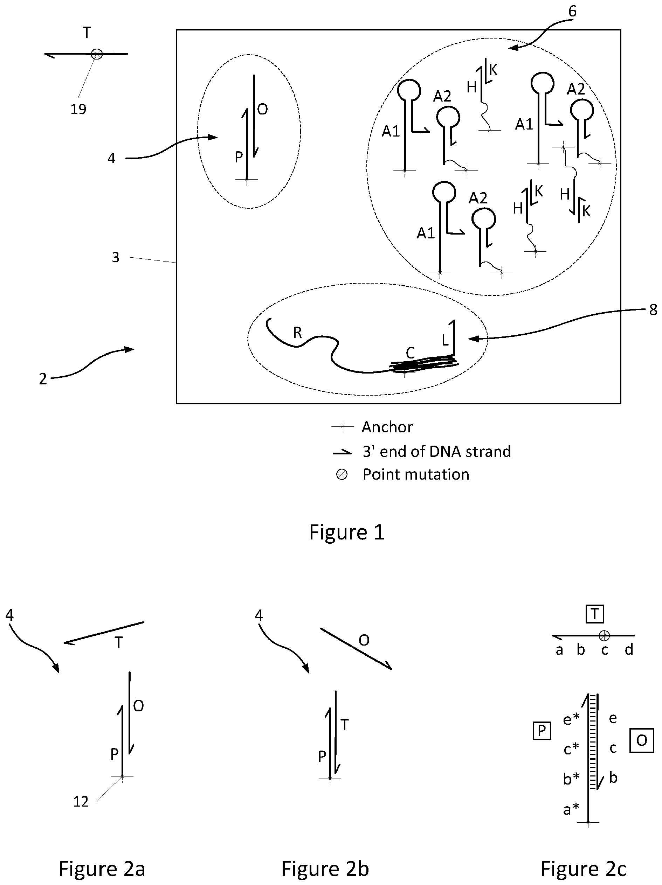

Figure 1 shows a DNA nano device 2 (also referred to as a nanorobot). The DNA nano device 2 is designed to allow early detection of cancer using a blood sample. The DNA nano device is sensitive enough to distinguish between circulating tumour DNA (ctDNA) 18 and cell-free DNA (cfDNA) which are both present in blood; the ctDNA target fragment 18 may merely carry a single point mutation 19 compared to the cfDNA. When ctDNA is detected the DNA nano device causes a signal that is observable to a user (e.g. a fluorescent signal), thus giving a rapid response to a clinician for diagnosis. Because the DNA nano device identifies specific genetic mutations which originate in tumour cells, it can provide insight into type and stage of the disease.

The DNA nano device 2 is formed of oligonucleotides - short, single-stranded molecules of DNA - through a self-assembly process, as is well known in the field.

The DNA nano device 2 includes a number of functional DNA formations that are anchored to a larger common DNA structure, in the illustrated example a rectangular body 3. The functional DNA formations can be between 10 and 90 base pairs long in some examples, but they may be longer (or shorter) as well - e.g. 100 base pairs long, 250 base pairs long, 300 base pairs long, 400 base pairs long, or more.

In the illustrations DNA strands that make up functional DNA formations are indicated by lines, with the 3' ends of DNA strands indicated with hooks, and anchor points of DNA formations to the body 3 are indicated with a symbol as indicated in the legend of Figure 1. The position of a point mutation on the target cfDNA strand is indicated with a symbol as indicated in the legend of Figure 1. In figures illustrating DNA strands in more detail groups of DNA bases, also referred to as domains, are represented as lower case letters, such as a, b, c, etc. Starred letters indicate complimentary domains - e.g. domain a* is complementary to domain a. Hybridisation of complementary domains is indicated in the illustration by an array of connecting lines between the complementary domains. Domains are a construct to aid in the understanding of the behaviour and interaction of DNA strands. The sequence of DNA bases in a domain may be given (e.g. for a specific target) or it may be selected to engineer the behaviour of a DNA strand (e.g. propensity to form a hairpin loop). In an example a domain can be between 5 and 50 base pairs long, for example 10 base pairs, 15 base pairs, 20 base pairs, 25 base pairs, 30 base pairs, or 40 base pairs long. Different domains on the same strand may have different lengths.

A wide variety of two- and three-dimensional shapes and forms can be used for the body 3 of the nano device 2, for example a tubular shape, an irregular shape or as illustrated a DNA sheet. The DNA nano device 2 includes a number of functional formations that are designed to interact with the environment (including the sample) and with each other. The functional formations are anchored to the body 3 at suitable positions. This can enable collocation of functional features that are intended to interact with each other, and facilitate their interaction. DNA is a particularly advantageous material for the body 3, as it can permit highly controllable anchoring of functional features at specific positions relative to each other. The functional formations include portions that can be released, exchanged, or accept other portions. This can enable interaction of functional features with the environment and each other. By virtue of the collocation and the ability to incorporate formations performing different functions, the molecular events resulting in an observable signal can be engineered to optimise performance of the DNA nano device 2.

The DNA nano device 2 illustrated in Figure 1 includes three functional portions or modules:
- detector 4,
- amplifier 6 and
- responder 8.

Each module performs a specific function, and by interacting they perform the intended detection and signalling.

The functional DNA formations interact according to the principles of competitive hybridisation to accept and release one another spontaneously, notably by way of toehold-mediated strand displacement and toehold exchange. Some of the functional DNA formations include portions with bistable configurations and can change configuration in response to interactions with one another (this is also referred to as molecular logic).

The detector module 4 and the amplifier module 6 are based on DNA computing principles such as toehold-mediated strand displacement, toehold exchange and molecular logic gates (molecules that perform a logical operation based on one or more physical or chemical inputs and a single output).

The responder 8 is based on dynamic DNA nanotechnology principles (DNA origami) and a cell-free expression system for producing a fluorescent signal.

The individual modules and their interactions are now described in more detail.

Figures 2a to 2c show the detector 4 in more detail in different states of operation. Figure 2a and 2b show a detector 4 before and after exposure to a target T, and Figure 2c shows the DNA strands of the detector 4 in more detail.

The detector 4 comprises a DNA strand that acts as probe P. The probe P is anchored to the body 3 of the DNA nano device 2 at an anchor point 12 (the illustrations generally show anchor points with a symbol as indicated in the legend of Figure 1). The probe P in Figure 2a is shown before exposure to the target T (the ctDNA sequence to be detected). In the initial configuration an output strand O, also referred to as a 'trigger' DNA strand or DNA sequence is (partially) hybridised to the probe strand P.

In Figure 2b the target T has displaced the output O by way of toehold-mediated strand displacement. The target T is hybridised to the probe P and the output O is released.

In use, the target DNA strand T attaches to the toehold region of the probe P and outcompetes the output strand O to displace it from the probe P.

The probe P and the output O are designed such that the probe P can distinguish between the target T ctDNA (carrying cancer mutations) and cfDNA. The probe P is complementary to the target T, whereas the output O has higher affinity to the probe than cfDNA, but has lower complementarity than the target T.

The output O triggers the downstream module, the amplifier 6.

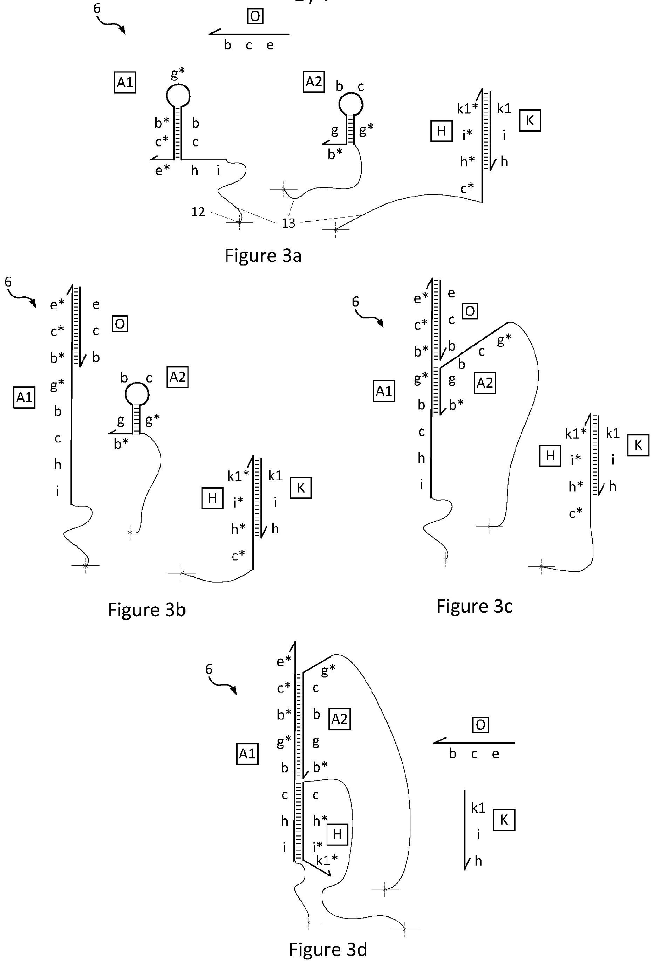

Figures 3a, 3b, 3c and 3d show a subunit of an amplifier 6 in more detail in different states of operation.

The illustrated amplifier 6 subunit includes three DNA strands: two strands that act as amplifiers A1 and A2 and a third strand that acts as a key holder H. Both the amplifiers A1, A2 and the key holder H are tethered to the body 3 of the DNA nano device 2 by way of tethers 13 that are attached to the body 3 at anchor points 12.

In Figure 3a the amplifier 6 subunit is shown in its initial configuration before exposure to the output O. In the initial configuration a key DNA strand K is hybridised to the key holder H. The two amplifier strands A1, A2 each include a molecular logic gate; that is, each has a hairpin loop metastable configuration that can be opened or closed depending on the circumstances.

In Figure 3b the amplifier 6 subunit is shown after exposure to the output O (from the detector module 4). The trigger strand O facilitates the conformational change of the logic gate of the first amplifier strand A1, that is O hybridises to A1, thereby changing the configuration (opening the hairpin loop) of A1.

In Figure 3c the amplifier 6 subunit is shown at a further stage of the amplification procedure. The exposed portion of A1 then hybridises to A2, during which the hairpin loop of A2 opens and A2 displaces O. Subsequently or concurrently, H hybridises to A1 and K is thereby released.

Figure 3d shows the amplifier 6 subunit where the second amplifier strand A2 and the key holder strand H are both hybridised to the first amplifier strand A1, and the key K and the trigger strand O have been released.

As a result of the operation of the amplifier 6 subunit two strands are released: a single key strand K and the original trigger strand O. The trigger strand O is then recycled to another similar amplifier 6 subunit. The released output O continues to a further amplifier subunit in the initial condition as shown in Figure 3a and by the analogous sequence of steps at that subunit a further key K 28 is released as well as the output strand O.

The keys K activate the downstream module, the responder 8.

In a catalytic fashion a single input O can be reused to release multiple key strands K from multiple amplifier subunits. Each amplifier 6 subunit takes an output O, and outputs a key K as well as an output O for further consumption. In the example illustrated in Figure 1 three amplifier 6 subunits are shown (each comprising two amplifier strands and a key holder), but any number of subunits may be included in the amplifier 6. The number of amplifier subunits can be varied to vary the amplification desired, that is the number of key strands released for each activating strand (output O from the detector). By inclusion of n amplifier subunits, n keys K are released. An initial single output strand O is amplified to n key strands K. This allows for detection of low concentrations of the target ctDNA by providing an amplified response in the form of n key strands K for a single target strand T.

Other specific amplification processes (generally known as hairpin DNA cascade amplification) may be used to produce multiple key strands catalytically from a single trigger strand (output strand O from the detector).

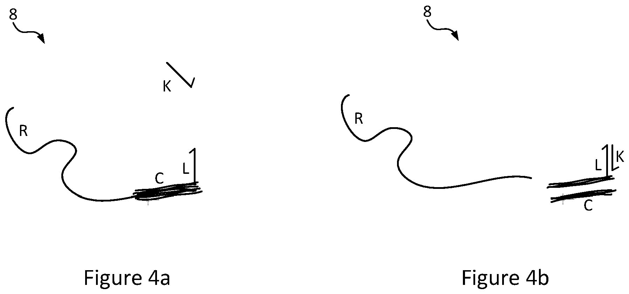

Figures 4a and 4b show a responder 8 in more detail in different states of operation. Figure 4a shows a responder 8 before exposure to a key K.

In the initial configuration shown in Figure 4a the illustrated responder 8 includes a capturing box C and a reporter gene R captured by the capturing box C.

The capturing box C includes a number of DNA strands which can form an enclosure that constrains the reporter gene R and prevents exposure of the enclosed portion. The enclosed portion of the reporter gene is a promoter sequence, so expression of the reporter gene is prevented until such time as the reporter gene R is released from the capturing box C and the promoter portion is exposed. The reporter gene R encodes a fluorescent protein such as green fluorescent protein, which can be produced by cell-free protein expression as is known in the art.

The capturing box C is a bistable DNA formation with a closed and an open configuration. The responder 8 includes a lock DNA strand L. Hybridisation of a key strand K to the lock strand L causes the capturing box C to undergo a change from the closed configuration into the open configuration.

In Figure 4b the key strand K is hybridised to the lock strand L, the capturing box C is in the open configuration and the reporter gene R is released.

The released reporter gene is expressed, and fluorescence of the expressed protein can indicate to an observer the presence of the target ctDNA in the sample.

The responder may include a number of instances of the capturing box C, each with reporter gene R. Each capturing box C can be opened by one of the key sequences K and thereby produce an observable fluorescent signal for a trace amount of analyte.

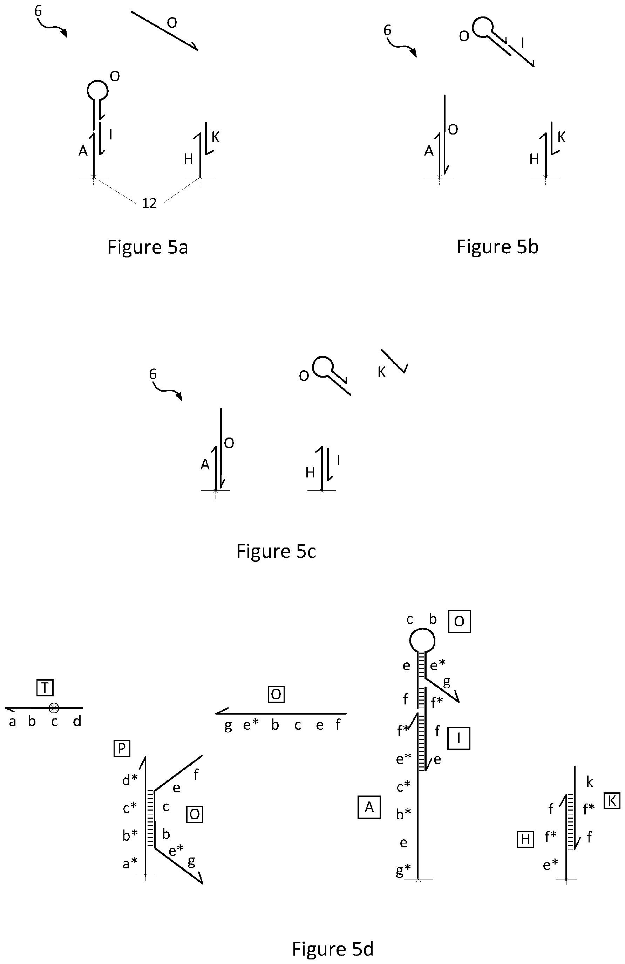

Figures 5a to 5d illustrate another amplifier 6 subunit where each amplification subunit includes an amplifier strand A and a key holder strand H, both anchored to the DNA body 3 at anchor points 12. Figures 5a-5c show the amplifier 6 subunit in different states of operation, and Figure 5d shows the DNA strands of the amplifier 6 subunit in more detail.

Figure 5a shows the initial configuration where two DNA strands are (partially) hybridised to the amplifier A: a further copy of the output strand O, and an intermediate DNA strand I. In the illustrated example the output strand O is in a hairpin loop metastable configuration.

In Figure 5b the output O has become hybridised to the amplifier A by way of toehold-mediated strand displacement. The output O attaches to the toehold region of the amplifier A and outcompetes the intermediate I to displace it from the amplifier A. The {intermediate I + output O} hybrid is released.

In Figure 5c the intermediate I has become hybridised to the key holder H by way of toehold-mediated strand displacement. The output O is released, as is the key K.

In the next step the released output O continues to a further amplifier subunit in the initial condition as shown in Figure 4a, and the output strand O is hybridised to a further amplifier A, thus displacing a further {intermediate I + output O} hybrid and by the analogous sequence of steps a further key K is released.

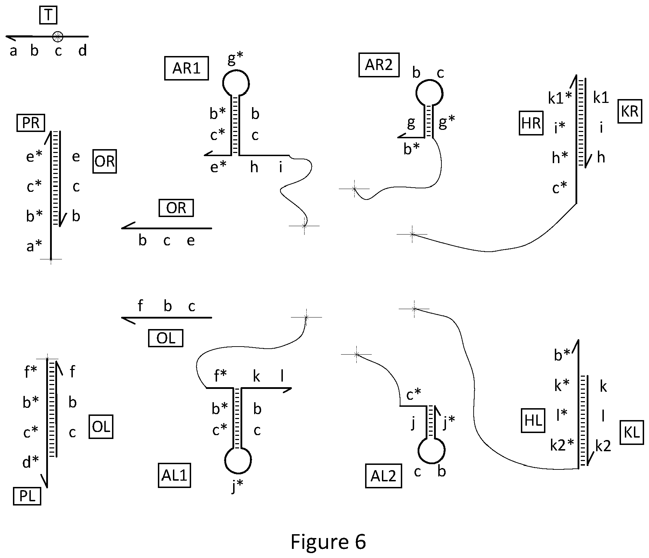

Figure 6 shows a variant of the amplifier subunit as described above with reference to Figures 3a to 3d. In this variant, the responder requires two keys, K1, K2 to operate (that is, change configuration from closed to open to release the reporter). The amplifier includes two pathways: a right-hand pathway and left-hand pathway (corresponding to the initial ctDNA being detected); for each pathway a probe PR, PL provides the appropriate trigger OR, OL. Because the responder requires two keys in this example high stability of the responder in the initial configuration can be enabled, and spontaneous activation of the responder can be prevented, thus enabling a low rate of false positives. In a related variant a single probe P carries two triggers, OR and OL, and both are released upon hybridisation of the target T to the probe P.

In a further variant both pathways (right and left) are adapted to amplify each other, creating a feed-forward loop. In this case the detection of a single target ctDNA molecule can cause a chain reaction, with the effect of high sensitivity and the response time of the DNA nano device being very short.

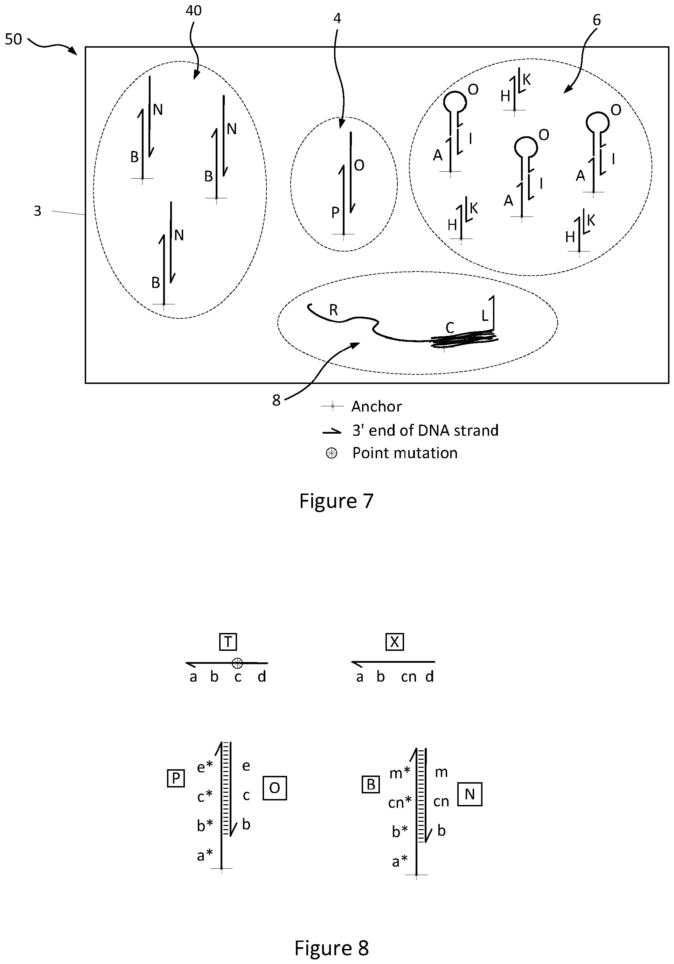

Figure 7 shows a variant of the DNA nano device described above. A DNA nano device 50 includes the detector 4, the amplifier module 6 and the responder 8 as described above. In addition the DNA nano device 50 includes a blocking portion (or module) 40 for capturing cfDNA (naturally present in blood) and releasing strands which are non-interacting with the rest of the device. The blocking module 40 includes a number of blocking probes B. The blocking probes B are similar to the probes P of the detector 4 described above, except that they hybridise best with interfering cfDNA strand X whereas the probe P hybridises best with the target ctDNA strand T.

Figure 8 shows a probe P, as described above with reference to the detector 8, and a blocking probe B in more detail. The blocking probe B is a DNA strand that is anchored to the body 3 of the DNA nano device 50 at an anchor point. In the initial configuration a non-interacting strand N is (partially) hybridised to the blocking probe B. An interfering strand X, such as a cfDNA strand, is hybridised to the blocking probe B and displaces a non-interacting strand N by way of toehold-mediated strand displacement. The non-interacting strand N released from the blocking probe B does not interact with the rest of the DNA nano device 50. By capturing interfering strands X, such as cfDNA, the blocking probes 40 can prevent non-target DNA strands X from causing unintended release of trigger DNA sequences O, and thereby reduce or prevent cross-talk and increase the specificity of the detector.

The blocking portion 40 may include a number of instances of the same species of blocking probe as illustrated in Figure 7, or the blocking portion 40 may include a number of different species of blocking probe (with one or more instances of each species of blocking probe).

In the examples illustrated above, the functional DNA strands that participate in the designed hybridisations are either anchored directly to the main DNA body 3 at an anchor point 12 (illustrated for example in Figure 2a), or they are anchored by way of a tether 13 connecting the functional DNA strands to the main body 3 at an anchor point 12 (as illustrated for example in Figure 3a). In a variant some functional DNA strands are anchored directly, some by way of a tether. Optionally some (or all) functional DNA strands may be provided untethered (e.g. the second amplifier strand A2 of an amplifier subunit), in free solution surrounding the DNA nano device. Direct anchoring gives good control over location and collocation different species of functional DNA strands in order to facilitate their interaction. Tethering gives less control over location, but it permits greater freedom of movement and change of conformation and interaction (hybridisation) with other functional DNA strands, and may provide better exposure of the functional DNA strands. Free solution provision may require relatively high concentrations to ensure sufficient availability. The tether may be a DNA sequence that is selected to discourage interaction with other parts of the system and to discourage formation of secondary structures such as hairpin loops. The tether may be of a material other than DNA such as a biopolymer (e.g. actin, lignin, tubulin). The tether length may be selected to give the functional DNA strand described above a certain range of movement.

In a variant the body to which the functional units (DNA strands) are attached is not a DNA structure as described above, but another material. For example the body may be a nanoparticle (organic or inorganic), a carbon nanotube, a micelle or a liposome. In a variant the body is a substrate (e.g. glass, silicon, polymer, ceramic) that may form part of a larger device such as a microfluidic device, a flow cell, a well plate, a cuvette, a test strip or other assay devices. Patterning of different functional units to a substrate may be with microfabrication techniques such as photolithography or soft lithography. Covalent bonding, for example, can anchor or tether the functional units to the body.

For designing a DNA nano device as described above a computer program product is used. The design of the DNA nano device is a multivariate problem and the exact sequence of DNA strands needed to build up a suitable DNA nano device depends on many factors. These include but are not limited to:
- The desired target DNA sequence, which is determined by the genetic mutations or biomarkers of interest for medical diagnostics or other purposes.
- The chemical stability of the nanorobot as a whole in the sample environment.
- The reaction rates
- The structure of the individual modules, i.e. the number of probes and amplifiers required for the desired sensitivity and the desired mechanism of transfer of output/inputs between them.
- Crosstalk minimisation: e.g. the specificity of the detector portion, how many and what structure of blocking probes, crosstalk between functional portions and units of the same DNA nano device, and also cross talk between different pathways (whether on a common DNA nano device or on separate DNA nano devices) intended to detect different targets. For example, a trigger of the type X (released by detecting ctDNA of type X) should only interact with amplifiers of type X. It should not interact with amplifiers of type Y and Z which indicate other type of mutations and possibly other types of cancer.

Performance of the DNA nano device can be optimised over these factors. A machine learning system is provided to perform such a multivariate optimisation and design the DNA nano device according to the desired characteristics and given a pool of cancer mutations to be detected.

The training data for the AI can be provided by simulation data, experimental data or both.

Figure 8 shows an example of a set of inputs, outputs, and the resulting DNA nano device 2. The software 62 simulates 60 the performance of the DNA nano device 2 in dependence on the inputs. For the optimum simulated DNA nano device 2 the software 50 outputs DNA sequences to form the DNA nano device, and potentially a feature layout and/or further manufacture information.

A DNA nano device 2 is generated as specified by the outputs. Properties of the DNA nano device 2 are tested and fed back to the software 50 to validate the simulation 60.

In the examples described above the DNA nano device is for in vitro analysis of a blood sample; it will be understood that the blood sample may undergo preparation, for example separation of the plasma component, DNA amplification, addition of reagents, or other processing. In a variant the DNA nano device can similarly be used with another bodily fluid or excretion such as lymph fluid, cerebrospinal fluid, pancreatic juice, bile juice, urine, saliva, sputum, stool, pleural fluid, amniotic fluid, tear fluid, or sweat. In a variant the DNA nano device can be used for in vivo detection.

In the examples described above the DNA nano device is for detection of ctDNA; in a variant the DNA nano device is adapted for detecting circulating tumour RNA.

In other examples the DNA nano device is adapted for detecting a circulating tumour protein, or other biomarkers such as alpha-feto protein, ion channel proteins or inflammatory markers. In this example the probe described above may be adapted to release a trigger DNA sequence on exposure to the biomarker, and the trigger DNA sequence causes the amplification and response to proceed as described above. In an example the detector described above with reference to Figure 1 includes a probe DNA sequence that is adapted (by selection of a suitable sequence) to form an aptamer capable of binding a target molecule such as a biomarker. An aptamer is a single-stranded oligonucleotide with a unique 3-dimensional structure and capable of binding targets (typically ligands) with high selectivity and specificity. The aptamer is selected such that binding to a target molecule causes a conformational change of the aptamer (for example a change in the secondary structure of the aptamer). The conformational change of the aptamer then causes release of a trigger DNA sequence, and the trigger DNA sequence causes the amplification and response to proceed as described above. In another variant the detector module of the DNA nano device includes, in addition to the probe DNA sequence P and trigger DNA sequence O hybrid as described with reference to Figure 1, an aptamer (optionally tethered to the DNA body) adapted to bind a target biomarker. In the initial configuration the aptamer does not bind to the probe P. The aptamer is adapted to change conformation upon binding the target biomarker, thus exposing a region that then hybridises to the probe P and thereby cause release of the trigger DNA sequence O from the probe P; the trigger DNA sequence then causes the amplification and response to proceed as described above.

In another example the DNA nano device is for detection of other trace DNA, RNA or other biomarkers, for example in forensic analysis.

While the examples described above refer to a DNA nano device for detecting a single ctDNA target species, in a variant a DNA nano device carries a library of probes, each for a different ctDNA species. The probes may all use the same pathway, or each probe may trigger a distinct pathway accommodated in the DNA nano device and may produce an observable signal specific to a probe type.

In the examples described above the DNA nano devices respond by way of a reporter gene for cell-free expression of a fluorescent protein. In variants other means of signalling may be implemented, for example by way of:
- fluorescence where a key K output at the amplification stage is adapted to displace a quencher from a suitable fluorescent tag;
- fluorescence resonance energy transfer (FRET) where a key K output at the amplification stage is adapted to include a FRET donor or acceptor molecule, and a reporter strand includes a complementary FRET acceptor/donor and is adapted to receive a key K strand;
- radioactive labelling, magnetic bead decoration, surface-enhanced Raman spectroscopy (SERS), gold or silver nanoparticle decoration

It will be understood that the present invention has been described above purely by way of example, and modifications of detail can be made within the scope of the invention.

Reference numerals appearing in the claims are by way of illustration only and shall have no limiting effect on the scope of the claims.

The term 'comprising' as used in this specification and claims preferably means 'consisting at least in part of'.

## Claims

1. An ensemble of DNA formations for detecting a biomarker, the formations including:
a detector adapted to accept a target biomarker and thereby to release a trigger DNA sequence by competitive hybridisation;
an amplifier adapted for hybridisation with the trigger DNA sequence and thereby to release a key DNA sequence and the trigger DNA sequence or a further trigger DNA sequence, wherein the release is by competitive hybridisation; and
a responder adapted for hybridisation with the key DNA sequence and thereby to cause a signal detectable to an observer to be produced.

2. An ensemble of DNA formations according to claim 1, wherein the competitive hybridisation is a toehold-mediated strand displacement or a toehold exchange.

3. An ensemble of DNA formations according to claim 1 or 2, wherein two or more of the formations are attached to a common body, preferably in a predetermined arrangement.

4. An ensemble of DNA formations according to any preceding claim, wherein the detector is adapted to accept a target DNA sequence, preferably circulating tumour DNA, and thereby to release a trigger DNA sequence; and/or wherein the detector is adapted for hybridisation with a plurality of biomarkers and thereby to release one or more trigger DNA sequences.

5. An ensemble of DNA formations according to any preceding claim, wherein the detector comprises one or more probe DNA sequences attached to a DNA body and adapted to hybridise with a target DNA sequence and release a trigger DNA sequence.

6. An ensemble of DNA formations according to any preceding claim, further comprising a blocker adapted to hybridise an interfering DNA sequence.

7. An ensemble of DNA formations according to any preceding claim, wherein the amplifier comprises a plurality of amplifier subunits, each amplifier subunit adapted to hybridise a trigger DNA sequence and to release a key DNA sequence and the trigger DNA sequence or a further trigger DNA sequence.

8. An ensemble of DNA formations according to any preceding claim, wherein the amplifier comprises one or more instances of one or more amplifier DNA sequences adapted to hybridise with a trigger DNA sequence; and one or more instances of a key holder DNA sequence adapted to release a key DNA sequence.

9. An ensemble of DNA formations according to Claim 8, wherein a first amplifier DNA sequence is adapted to change conformation upon hybridisation with the trigger DNA sequence.

10. An ensemble of DNA formations according to Claim 9, wherein a second amplifier DNA sequence is adapted to change conformation upon hybridisation with the first DNA sequence and thereby to release the trigger DNA sequence.

11. An ensemble of DNA formations according to any preceding claims, wherein the amplifier comprises a plurality of amplification pathways for release of different species of key DNA sequences.

12. An ensemble of DNA formations according to any preceding claim, wherein the responder comprises a DNA capture formation adapted at least partially to enclose a reporter in a first configuration, and to release the reporter in a second configuration.

13. A method of detecting a target biomarker comprising introducing an ensemble of DNA formations according to any preceding claim to a sample of bodily fluid *in vitro.*

14. A method of detecting a target biomarker, comprising:
hybridising a target biomarker to a probe and thereby releasing a trigger DNA sequence by competitive hybridisation;
hybridising the trigger DNA sequence to an amplifier DNA sequence and thereby causing one or more conformational changes of the amplifier DNA sequence, releasing a key DNA sequence, and releasing the or a trigger DNA sequence, wherein the releasing is by competitive hybridisation; and
hybridising the key DNA sequence to a lock DNA sequence portion of a DNA capture formation and thereby cause a signal detectable to an observer to be produced, preferably by changing configuration of the DNA capture formation to release a reporter.

15. A computer program product comprising software code adapted to generate, when executed, a simulation of the ensemble of DNA formations according to any of Claims 1 to 12.

## Patentansprüche

1. Ein Ensemble von DNA-Formationen zum Nachweis eines Biomarkers, wobei die Formationen Folgendes beinhalten:
einen Detektor, der angepasst ist, einen Target-Biomarker aufzunehmen und dadurch eine Trigger-DNA-Sequenz durch kompetitive Hybridisierung freizusetzen;
ein Amplifikationsmittel, das angepasst ist, mit der Trigger-DNA-Sequenz zu hybridisieren und somit eine Schlüssel-DNA-Sequenz und die Trigger-DNA-Sequenz oder eine weitere Trigger-DNA-Sequenz freizusetzen, wobei die Freisetzung durch kompetitive Hybridisierung erfolgt; und
ein Antwortmittel, das angepasst ist, mit der Schlüssel-DNA-Sequenz zu hybridisieren und somit die Produktion eines für einen Beobachter nachweisbaren Signals zu bewirken.

2. Ensemble von DNA-Formationen nach Anspruch 1, wobei die kompetitive Hybridisierung eine "Toehold"-vermittelte Strangverdrängung oder ein "Toehold"-Austausch ist.

3. Ensemble von DNA-Formationen nach Anspruch 1 oder 2, wobei zwei oder mehrere der Formationen an einem gemeinsamen Körper, bevorzugt in einer vorbestimmten Anordnung, hängen.

4. Ensemble von DNA-Formationen nach einem der vorhergehenden Ansprüche, wobei der Detektor angepasst ist, eine Target-DNA-Sequenz, bevorzugt zirkulierende Tumor-DNA, aufzunehmen und somit eine Trigger-DNA-Sequenz freizusetzen; und/oder wobei der Detektor angepasst ist, mit einer Vielzahl von Biomarkern zu hybridisieren und somit eine oder mehrere Trigger-DNA-Sequenzen freizusetzen.

5. Ensemble von DNA-Formationen nach einem der vorhergehenden Ansprüche, wobei der Detektor eine oder mehrere Sonden-DNA-Sequenzen beinhaltet, die an einem DNA-Körper hängen und angepasst sind, mit einer Target-DNA-Sequenz zu hybridisieren und eine Trigger-DNA-Sequenz freizusetzen.

6. Ensemble von DNA-Formationen nach einem der vorhergehenden Ansprüche, das ferner ein Blockierungsmittel beinhaltet, das angepasst ist, eine Interfering-DNA-Sequenz zu hybridisieren.

7. Ensemble von DNA-Formationen nach einem der vorhergehenden Ansprüche, wobei das Amplifikationsmittel eine Vielzahl von Amplifikationsmittel-Untereinheiten beinhaltet, wobei jede Amplifikationsmittel-Untereinheit angepasst ist, eine Trigger-DNA-Sequenz zu hybridisieren und eine Schlüssel-DNA-Sequenz und die Trigger-DNA-Sequenz oder eine weitere Trigger-DNA-Sequenz freizusetzen.

8. Ensemble von DNA-Formationen nach einem der vorhergehenden Ansprüche, wobei das Amplifikationsmittel Folgendes beinhaltet: eine oder mehrere Instanzen von einer oder mehreren Amplifikationsmittel-DNA-Sequenzen, die angepasst sind, mit einer Trigger-DNA-Sequenz zu hybridisieren; und eine oder mehrere Instanzen einer Schlüsselhalter-DNA-Sequenz, die angepasst sind, eine Schlüssel-DNA-Sequenz freizusetzen.

9. Ensemble von DNA-Formationen nach Anspruch 8, wobei eine erste Amplifikationsmittel-DNA-Sequenz angepasst ist, ihre Konformation nach Hybridisierung mit der Trigger-DNA-Sequenz zu ändern.

10. Ensemble von DNA-Formationen nach Anspruch 9, wobei eine zweite Amplifikationsmittel-DNA-Sequenz angepasst ist, ihre Konformation nach Hybridisierung mit der ersten DNA-Sequenz zu ändern und somit die Trigger-DNA-Sequenz freizusetzen.

11. Ensemble von DNA-Formationen nach einem der vorhergehenden Ansprüche, wobei das Amplifikationsmittel eine Vielzahl von Amplifikationswegen zur Freisetzung verschiedener Spezies von Schlüssel-DNA-Sequenzen beinhaltet.

12. Ensemble von DNA-Formationen nach einem der vorhergehenden Ansprüche, wobei das Antwortmittel eine DNA-Capture-Formation beinhaltet, die angepasst ist, einen Reporter in einer ersten Konfiguration mindestens teilweise zu umschließen und den Reporter in einer zweiten Konfiguration freizusetzen.

13. Ein Verfahren zum Nachweis eines Target-Biomarkers, das das Einführen eines Ensembles von DNA-Formationen nach einem der vorhergehenden Ansprüche in eine Körperflüssigkeitsprobe in vitro beinhaltet.

14. Verfahren zum Nachweis eines Target-Biomarkers, das Folgendes beinhaltet:
Hybridisieren eines Target-Biomarkers an eine Sonde und somit Freisetzen einer Trigger-DNA-Sequenz durch kompetitive Hybridisierung;
Hybridisieren der Trigger-DNA-Sequenz an eine Amplifikationsmittel-DNA-Sequenz und somit Bewirken einer oder mehrere Konformationsänderungen der Amplifikationsmittel-DNA-Sequenz, Freisetzen einer Schlüssel-DNA-Sequenz und Freisetzen der oder einer Trigger-DNA-Sequenz, wobei das Freisetzen durch kompetitive Hybridisierung erfolgt; und
Hybridisieren der Schlüssel-DNA-Sequenz an einen Schloss-DNA-Sequenzabschnitt einer DNA-Capture-Formation und somit Bewirken der Produktion eines für einen Beobachter nachweisbaren Signals, bevorzugt durch Änderung der Konfiguration der DNA-Capture-Formation, um einen Reporter freizusetzen.

15. Ein Computerprogrammprodukt, das Softwarecode beinhaltet, der, wenn er ausgeführt wird, dazu angepasst ist, eine Simulation des Ensembles von DNA-Formationen nach einem der Ansprüche 1 bis 12 zu generieren.

## Revendications

1. Ensemble de formations d'ADN pour la détection d'un biomarqueur, les formations comprenant :
un détecteur conçu pour recevoir un biomarqueur cible en libérant ainsi une séquence d'ADN d'initiation par hybridation compétitive ;
un amplificateur conçu pour une hybridation avec la séquence d'ADN d'initiation en libérant ainsi une séquence d'ADN clé et la séquence d'ADN d'initiation ou une séquence d'ADN d'initiation supplémentaire, la libération ayant lieu par hybridation compétitive ; et
un répondeur conçu pour une hybridation avec la séquence d'ADN clé en provoquant ainsi la production d'un signal détectable par un observateur.

2. Ensemble de formations d'ADN selon la revendication 1, dans lequel l'hybridation compétitive est un déplacement de brin médié par la région toehold (TMSD) ou un échange de région toehold.

3. Ensemble de formations d'ADN selon la revendication 1 ou 2, dans lequel deux ou plusieurs des formations sont fixées à un corps commun, de préférence selon une disposition prédéterminée.

4. Ensemble de formations d'ADN selon l'une quelconque des revendications précédentes, dans lequel le détecteur est conçu pour recevoir une séquence d'ADN cible, de préférence de l'ADN tumoral circulant, en libérant ainsi une séquence d'ADN d'initiation ;
et/ou dans lequel le détecteur est conçu pour une hybridation avec une pluralité de biomarqueurs en libérant ainsi une ou plusieurs séquences d'ADN d'initiation.

5. Ensemble de formations d'ADN selon l'une quelconque des revendications précédentes, dans lequel le détecteur comprend une ou plusieurs séquences sondes d'ADN fixées à un corps d'ADN et conçues pour s'hybrider avec une séquence d'ADN cible et libérer une séquence d'ADN d'initiation.

6. Ensemble de formations d'ADN selon l'une quelconque des revendications précédentes, comprenant en outre un bloqueur conçu pour s'hybrider avec une séquence d'ADN interférent.

7. Ensemble de formations d'ADN selon l'une quelconque des revendications précédentes, dans lequel l'amplificateur comprend une pluralité de sous-unités d'amplification, chaque sous-unité d'amplification étant conçue pour s'hybrider avec une séquence d'ADN d'initiation et pour libérer une séquence d'ADN clé et la séquence d'ADN d'initiation ou une séquence d'ADN d'initiation supplémentaire.

8. Ensemble de formations d'ADN selon l'une quelconque des revendications précédentes, dans lequel l'amplificateur comprend une ou plusieurs occurrences d'une ou plusieurs séquences d'ADN d'amplification conçues pour s'hybrider avec une séquence d'ADN d'initiation ; et une ou plusieurs occurrences d'une séquence d'ADN porte-clé conçue pour libérer une séquence d'ADN clé.

9. Ensemble de formations d'ADN selon la revendication 8, dans lequel une première séquence d'ADN d'amplification est conçue pour changer de conformation lors de l'hybridation avec la séquence d'ADN d'initiation.

10. Ensemble de formations d'ADN selon la revendication 9, dans lequel une deuxième séquence d'ADN d'amplification est conçue pour changer de conformation lors de l'hybridation avec la première séquence d'ADN en libérant ainsi la séquence d'ADN d'initiation.

11. Ensemble de formations d'ADN selon l'une quelconque des revendications précédentes, dans lequel l'amplificateur comprend une pluralité de voies d'amplification pour la libération de différentes espèces de séquences d'ADN clés.

12. Ensemble de formations d'ADN selon l'une quelconque des revendications précédentes, dans lequel le répondeur comprend une formation de capture d'ADN conçue au moins en partie pour enfermer un rapporteur dans une première configuration, et pour libérer le rapporteur dans une deuxième configuration.

13. Procédé de détection d'un biomarqueur cible comprenant l'introduction d'un ensemble de formations d'ADN selon l'une quelconque des revendications précédentes dans un échantillon de fluide corporel *in vitro.*

14. Procédé de détection d'un biomarqueur cible, comprenant :
l'hybridation d'un biomarqueur cible avec une sonde en libérant ainsi une séquence d'ADN d'initiation par hybridation compétitive ;
l'hybridation de la séquence d'ADN d'initiation avec une séquence d'ADN d'amplification en provoquant ainsi une ou plusieurs modifications de la conformation de la séquence d'ADN d'amplification, en libérant une séquence d'ADN clé, et en libérant la ou une séquence d'ADN d'initiation, la libération ayant lieu par hybridation compétitive ; et
l'hybridation de la séquence d'ADN clé avec une partie correspondant à une séquence d'ADN de verrouillage d'une formation de capture d'ADN en provoquant ainsi la production d'un signal détectable par un observateur, de préférence en modifiant la configuration de la formation de capture d'ADN pour libérer un rapporteur.

15. Produit sous forme de programme informatique comprenant un code logiciel conçu pour générer, lors de son exécution, une simulation de l'ensemble de formations d'ADN selon l'une quelconque des revendications 1 à 12.
